# EUROPEAN PATENT APPLICATION

(11) **EP 4 822 707 A1**
(43) Date of publication of application: **14.10.2026**
(21) Application number: 25169835.3
(22) Date of filing: 10.04.2025
(51) Int. Cl.: G16H 30/40, A61B 5/00, G16H 50/20

(54) **A METHOD FOR MEASURING EXUDATE SPREAD IN A WOUND DRESSING**

(71) Applicant: Mölnlycke Health Care AB, 431 21 Mölndal (SE)
(72) Inventor: JAKOBSSON, Conny, 443 38 Lerum (SE)
(74) Representative: Kransell & Wennborg KB

(57) **Abstract**

The present disclosure relates to a computer-implemented method, system, and corresponding computer program product for measuring exudate spread in a wound dressing. The disclosed solution involves acquiring an image of the wound dressing and obtaining a size parameter from an identifier associated with the dressing, whereby the size parameter and the image are subsequently used to measure the exudate spread within the dressing.

## Description

### TECHNICAL FIELD

The present disclosure relates to a computer-implemented method, system, and corresponding computer program product for measuring exudate spread in a wound dressing. The disclosed solution involves acquiring an image of the wound dressing and obtaining a size parameter from an identifier associated with the dressing, whereby the size parameter and the image are subsequently used to measure the exudate spread within the dressing.

### BACKGROUND

In medical wound care, the assessment of wound exudate, including fluids produced by wounds as part of the healing process, is essential for monitoring healing and identifying potential complications. Accurate measurement and tracking of exudate spread within wound dressings can provide critical insights for healthcare professionals, including indications of infection, the need for dressing changes, and overall wound health.

Traditionally, exudate assessment relies on manual inspection, where healthcare providers visually examine the wound dressing to estimate exudate levels and spread. Such an approach is often subjective, time-consuming, and may require removing the dressing from the wound. Such disruption to the wound environment can increase infection risks and interfere with the healing process. Moreover, manual methods may lead to inconsistent assessments, especially when performed by different practitioners or across varying clinical settings.

While some advancements have been made, including the use of moisture sensors and absorbent materials in wound dressings, existing techniques often fall short in providing precise, objective, and consistent measurements of exudate spread. Most methods are either invasive or limited to providing only general information about fluid presence, without offering a detailed analysis of exudate distribution within the dressing.

Taking the above into account, there seems to be room for further improvements in relation to non-invasive and accurate methods for assessing exudate spread within wound dressings. Developments in digital imaging, data processing, and related technologies could enable more objective and consistent measurements of exudate distribution, reducing subjectivity and minimizing wound disturbance. Such advancements would support healthcare professionals in making more informed and timely decisions in wound care management.

### SUMMARY

In view of the above-mentioned and other drawbacks of the prior art, it is an object of the present disclosure to provide improvements in relation to non-invasive and accurate measurement of exudate spread within wound dressings, based on acquired images and relevant dressing data.

According to an aspect of the present disclosure, it is therefore provided a computer-implemented method for measuring exudate spread in a wound dressing, the method comprising the steps of acquiring, using a processing unit, an image of a wound dressing, acquiring, using the processing unit, a size parameter of the wound dressing based on data obtained from an identifier associated with the dressing, determining, using the processing unit, a scale for exudate measurement based on the size parameter, identifying, using the processing unit, an exudate area within the acquired image, and calculating, using the processing unit, a spread of exudate in the wound dressing based on the exudate area and the determined scale.

The present disclosure enables non-invasive and objective measurement of exudate spread in wound dressings by combining image acquisition with size data obtained from the wound dressing. Specifically, the core idea is to integrate image processing with coded size data related to the wound dressing to measure exudate spread more precisely. By automating the exudate measurement, the method thus removes the need for manual inspection. Advantages following such an implementation includes improved consistency and accuracy in exudate assessment, as the size parameter acquired through the identifier reduces variability in measurements across different healthcare professionals. Continuous tracking of exudate spread may also be achieved without removing the dressing, preserving the wound environment and minimizing infection risks.

The method according to the present disclosure thus enhances wound care management by improving measurement precision and supporting consistent assessments, potentially promoting better healing outcomes. An implementation of the present disclosure may for example utilize the calculated spread of exudate to support downstream decision-making processes, such as suggesting dressing changes, updating patient treatment protocols, or triggering specific clinical workflows. The calculated spread may also be compared against reference values for particular wound dressing types or wound profiles, thereby enabling the solution according to the present disclosure to provide real-time decision support or generate context-specific recommendations. Accordingly, the calculated spread of exudate is not generally intended to merely be observed or displayed. Rather, the calculated spread of exudate is generally to be actively used to guide clinical actions.

Preferably, the size parameter may define a pixel-to-distance ratio or calibration factor that enables the processing unit to convert pixel measurements of the exudate area into real-world units such as square centimeters. Such a transformation allows for objective quantification and comparison across different dressing types and image resolutions. The exudate area may for example be determined by counting the number of pixels within a defined region in the image corresponding to the exudate, with the pixel count converted into a real-world area value based on the calculated scaling factor.

Within the context of the present disclosure, the expression "size parameter" should be interpreted broadly, and may refer to any data that allows the processing unit to determine the physical scale of the wound dressing or parts thereof. In some embodiments, the size parameter may define a known width, height, or area of the dressing itself, typically corresponding to the visible absorbent body or absorption region of the dressing. In other embodiments, the size parameter may refer to the dimensions of a predefined portion of the dressing, such as a reference marker or identifier. The size parameter may be obtained directly from digital data encoded within an identifier, for example a machine-readable code as is discussed below, or may be derived visually when the identifier has a known physical size and is visible in the acquired image. In the latter case, the identifier may function as a scale reference, enabling the system to determine a pixel-to-distance ratio by comparing the known dimensions of the identifier to its appearance in the image. The same identifier may also encode other relevant metadata, such as the absorption capacity of the dressing, product type, or reference to an external data source. By accommodating both digitally encoded and visually inferred size information, the present disclosure allows flexible integration of scale calibration within the image-based analysis workflow, while maintaining compatibility with different types of dressings and identifier formats.

In one embodiment, the identifier is a machine-readable code, such as for example selected to be one of a QR code, barcode, or an NFC tag. By using a machine-readable code, the process of acquiring the size parameter of the wound dressing can be streamlined and automated, reducing the need for manual data entry. Such an approach also allows the code to contain or reference multiple parameters relevant to the dressing, including size, absorption characteristics, and product type. When integrated with imaging-based analysis, the machine-readable code ensures that the acquired data corresponds exactly to the specific dressing in use, supporting the accuracy of the exudate spread calculation. An additional advantage is the possibility to use widely available scanning technologies, such as smartphone cameras or NFC-capable devices, which allows the solution to be deployed without requiring specialized hardware.

Preferably, the machine-readable code is provided on an outer surface of the wound dressing, the outer surface being away from the wound. Placement of the code on the exposed side of the wound dressing enables it to be accessed or scanned without removing the dressing from the wound, thereby supporting a non-invasive workflow. The selected position is particularly important in clinical environments, where disturbing the dressing may compromise sterility or delay healing.

In some embodiments, the image of the wound dressing is acquired using an imaging device communicatively connected to the processing unit. The imaging device may be integrated in, or externally connected to, a handheld electronic device, such as a smartphone or tablet. Preferably, the acquired image is not limited to a standard visual representation but may include enhanced image content, such as overlays, annotations, or metadata linked to the imaging conditions or the detected wound area. For example, certain implementations may apply contrast adjustment, edge detection, or illumination correction to improve accuracy in identifying the exudate area. By acquiring enhanced images, the reliability of the exudate spread analysis is improved, particularly under varying environmental lighting or dressing material conditions. Furthermore, connecting the imaging device to the processing unit ensures that the image can be directly processed without the need for manual transfer or formatting.

It may further be possible to evaluate image quality parameters or contextual metadata, such as lighting conditions, angle of acquisition, or dressing type, to estimate a confidence value associated with the calculated exudate spread. Accordingly, the confidence values may be used to trigger re-imaging prompts, flag low-certainty results for manual review, or weight measurements during longitudinal analysis. Such an implementation contributes to improved measurement reliability and allows for dynamic adaptation in real-world clinical settings.

In line with the present disclosure, it may also be possible to, in some embodiments, prompt a user to reacquire an image when the confidence value associated with the measurement falls below a predefined threshold. Such feedback may be provided through a user interface, guiding the user to adjust imaging conditions or verify framing. By such an implementation it may be possible to further improve measurement reliability while maintaining ease of use in clinical workflows.

Preferably, the method according to the present disclosure may also further comprise the step of acquiring, using the processing unit, an absorption capacity parameter of the wound dressing based on the identifier, wherein the calculated spread of exudate is correlated with the absorption capacity parameter. The absorption capacity parameter may provide a quantitative reference that allows the measured exudate to be contextualized. For example, a high spread value within a high-capacity dressing may be considered acceptable, while the same value in a low-capacity dressing may indicate a risk of saturation. By incorporating such a parameter into the analysis, it may be possible to enable more clinically relevant assessments and supports timely intervention decisions, such as replacing a dressing before failure or leakage occurs.

In some embodiments, the absorption capacity parameter or other metadata associated with the identifier may further be used to define one or more virtual borders within the wound dressing. A virtual border may represent a boundary beyond which the spread of exudate is considered undesirable. For example, a virtual border may correspond to the border (edges) of the absorbent body of a dressing, i.e. an area defined by a virtual border may essentially correspond to an area of the absorbent body of a dressing. The virtual border may be calculated or retrieved from external dressing data and subsequently rendered as an overlay within the graphical user interface (GUI) of the electronic user device. The user may then assess the position of the exudate spread relative to this virtual border, either manually or with support from the processing unit. In some implementations, the system may automatically trigger an alert when the spread is determined to be approaching or extending beyond the virtual border, indicating the need for dressing replacement or further clinical action.

Additionally, when the identifier is visible and possesses a known geometry or layout, such as for example in the case of a machine-readable code printed in a fixed position at the wound dressing, it may also be used to determine the orientation of the dressing relative to gravity. Furthermore, the known geometry may also be used to determine the orientation of the dressing relative to gravity. Such orientation awareness enables directional analysis of the exudate spread, which may be particularly relevant for vertical wound sites such as legs or arms, where gravitational effects can cause exudate to accumulate preferentially toward the lower edge of the dressing. By comparing the detected spread pattern with the known dressing orientation, the system may apply directional thresholds or context-specific virtual borders that reflect the expected fluid dynamics. Such an implementation according allows the solution to support more precise and clinically meaningful interpretations of the spread distribution, improving reliability of alerts and recommendations.

Additionally, in some embodiments, the method according to the present disclosure is selected to further comprise the step of monitoring the exudate spread over time by periodically acquiring images of the wound dressing and comparing the calculated spread of exudate in the periodically acquired images. Such temporal tracking allows healthcare professionals to observe progression or regression of the wound condition without disturbing the dressing, which is particularly valuable for detecting early signs of complications or confirming positive healing trends. Accordingly, it may be possible to store and align historical image data for trend analysis, enabling longitudinal monitoring that supports evidence-based wound care decisions.

In a further embodiment, the method according to the present disclosure may comprise the step of framing the exudate area within the acquired image, wherein the framing is performed either manually by a user or automatically using the processing unit. Manual framing may allow a healthcare professional to mark the region of interest directly on the image (e.g. using known framing tools such as a square or lasso tool), while automatic framing enables the solution according to the present disclosure to identify the relevant area based on predefined image analysis procedures. By supporting both modes of operation, the solution according to the present disclosure accommodates different clinical workflows and user preferences, while ensuring that the framing step is consistent and reproducible. Framing the exudate area also serves as a preparatory step for the subsequent spread calculation, improving the reliability of the measurement output. The step of framing may in some embodiments be performed as part or a sub-step of identifying the exudate area within the acquired image, such as when the framing is performed semi-automatically or fully automatically.

Still further, in some embodiments, the step of framing the exudate area within the acquired image is enhanced by using an artificial intelligence (AI) based algorithm trained to identify edges or contrast boundaries indicative of exudate spread. Such enhancement allows the solution according to the present disclosure to adapt to different wound dressing types, lighting conditions, and exudate appearances. The AI-based approach may detect subtle or irregular spread patterns that would be difficult to identify using rule-based techniques or manual observation. Through continuous improvement of the algorithm, the accuracy and robustness of the automatic framing process may be further increased over time.

Furthermore, in some embodiments, the method according to the present disclosure may be selected to further comprise the steps of generating, using the processing unit, an alert for a healthcare professional when a threshold level of exudate spread is detected based on the calculated spread of exudate, and providing, using the processing unit, the alert within the GUI of the electronic user device. The alert may in some embodiments be configured to prompt timely intervention, such as replacement of the dressing, clinical evaluation, or escalation of care. Displaying the alert directly within the mentioned GUI ensures that relevant information is presented in an accessible and actionable format at the point of care, supporting fast and informed decision-making.

Furthermore, the alert mechanism may additionally include a quantitative representation of the exudate spread relative to the maximum absorption capacity of the wound dressing. For example, the GUI may be configured to display a percentage value indicative of how much of the dressing's capacity has been utilized, based on the correlation between the calculated spread of exudate and the known absorption capacity parameter. Such a representation allows the user to interpret the urgency of the situation with greater clarity, particularly in cases where the dressing is nearing saturation. The visual presentation of this capacity percentage may be augmented with color coding, threshold markers, or progress bar-style elements, thereby enabling an intuitive assessment at a glance, which possibly may be beneficial in clinical settings where fast and reliable interpretation is required, as well as in remote care or patient-managed environments where professional supervision is limited.

In one embodiment, the alert is customized based on a threshold level set according to a wound type, a wound dressing type, or a medical history of the patient provided with the wound dressing. By enabling customization of alert thresholds, the solution according to the present disclosure may account for clinical context and patient-specific factors, ensuring that alerts are meaningful and clinically relevant. For instance, a higher threshold may be appropriate for highly absorbent dressings, while patients with a known history of infection risk may benefit from more conservative thresholds. Such an adaptive behavior may possibly improve the specificity of the alert and reduces the occurrence of unnecessary or irrelevant notifications.

Possibly, the interpretation of the calculated exudate spread may be configured based on wound-specific or patient-specific data. For example, wounds located on high-friction areas or in regions with limited air exposure may have different acceptable spread thresholds. As a concrete example, patients with diabetes may benefit from more conservative criteria.

Additionally, the method further may comprise the step of analyzing, using the processing unit, additional wound characteristics selected from redness, swelling, or signs of infection based on data extracted from the acquired image. Such visual indicators may be assessed in parallel with the exudate analysis to provide a more comprehensive understanding of wound condition. By incorporating such secondary parameters, the solution according to the present disclosure may contribute to early detection of complications and provide more nuanced information to healthcare professionals, supporting a broader clinical assessment without requiring removal of the dressing.

Preferably, the identifier provides a pointer to an external source containing at least one of the size parameters and the absorption capacity parameter of the wound dressing. Such a configuration enables the solution according to the present disclosure to retrieve dressing-specific data from a centralized or remotely maintained source, reducing the need for locally stored or pre-encoded information. Such an implementation allows manufacturers to maintain and update product specifications independently of the scanning or imaging device, and also facilitates the use of generic identifiers that resolve to detailed metadata at runtime. The result may be a more flexible and scalable solution, with improved maintainability and integration potential.

It may further be possible to arrange the method according to present disclosure to further comprise the step of detecting, using the processing unit, a color change in the exudate area within the acquired image as an indicator of infection or wound deterioration. Colorimetric analysis may reveal changes in wound fluid characteristics, such as a shift from serous to purulent exudate, or the presence of discoloration indicating inflammation or tissue breakdown. By automatically detecting such changes, the solution according to the present disclosure may support early identification of clinical deterioration, prompting intervention before more serious complications develop. The ability to detect color changes without removing the dressing enhances the value of image-based monitoring and contributes to proactive wound care.

Additionally, it may in some embodiments be possible to arrange the method according to the present disclosure to further comprise the steps of storing the acquired images and calculated exudate spread values in a patient record database for continuous monitoring. Maintaining a record of visual and quantitative data over time allows healthcare providers to access historical wound data during follow-up visits or remote consultations. Such documentation improves continuity of care and supports collaborative decision-making among multidisciplinary teams. In addition, stored data may serve as a basis for retrospective analysis or quality assurance processes within clinical environments.

In a further embodiment, the method additionally comprises the step of generating a trend analysis report over time, based on the calculated spread of exudate and additional wound characteristics. Such a report may provide insights into wound healing or progression, highlighting patterns that are not apparent from isolated observations. For example, a gradual increase in exudate levels or worsening of secondary indicators such as redness may signal emerging complications. Conversely, stable or improving metrics may confirm treatment efficacy. By presenting trends in a structured format, the solution according to the present disclosure may enhance clinical decision support and enable data-driven wound management strategies.

According to another aspect of the present disclosure, there is provided a computer system for measuring exudate spread in a wound dressing, wherein the computer system comprises a processing unit adapted to acquire an image of a wound dressing, acquire a size parameter of the wound dressing based on data obtained from an identifier associated with the dressing, determine a scale for exudate measurement based on the size parameter, identify an exudate area within the acquired image, and calculate a spread of exudate in the wound dressing based on the exudate area and the determined scale. This aspect of the present disclosure provides similar advantages as discussed above in relation to the previous aspects of the present disclosure.

In some embodiments, the computer system comprises a portable electronic user device, such as a smartphone or tablet, operatively connected to a remote processing server via a communication network. The portable electronic user device may be configured to acquire the image of the wound dressing and transmit the acquired image, together with associated identifier data, to the remote processing server for further processing. Communication between the user device and the server may be established over a wireless network, such as a Wi-Fi or cellular connection, using for example standard communication protocols.

In such configurations, the remote server may be adapted to perform computational steps including determining the scale based on the received size parameter, identifying the exudate area within the received image, and calculating the spread of exudate. By offloading these steps to a server, the solution according to the present disclosure may reduce computational demands on the user device, allowing for implementation on lower-power hardware. Server-side processing may further enable more resource-intensive operations, such as execution of AI-based framing algorithms or access to centralized dressing specification databases.

Preferably, feedback based on the calculated exudate spread, including for example a visual overlay indicating the framed exudate region, a numerical result, or an alert notification, etc. may be transmitted from the server back to the user device for presentation within a graphical user interface (GUI) for example provided at a display unit of the user device. The GUI may also be configured to provide user guidance, display confidence values or alert thresholds, or prompt the user to retake the image if image quality is deemed insufficient. Such a client-server architecture enables real-time, interactive use of the system while maintaining flexibility in deployment and system scaling.

Furthermore, the computer system may also be configured to store the acquired images and calculated exudate spread values in a patient record database, for example provided locally at a hospital or in relation to the above-mentioned server. Such functionality supports continuous documentation and historical tracking of wound development, allowing for longitudinal assessment across multiple treatment sessions. Stored data may be accessed by clinicians for follow-up, remote consultations, or quality assurance, thereby improving continuity and coordination of care.

According to a further aspect of the present disclosure, there is provided a computer program comprising instructions which, when executed by a computer or processor, cause a computer system to measure exudate spread in a wound dressing, wherein the computer system comprises a processing unit, and wherein the computer program comprises code for acquiring, using a processing unit, an image of a wound dressing, code for acquiring, using the processing unit, a size parameter of the wound dressing based on data obtained from an identifier associated with the dressing, code for determining, using the processing unit, a scale for exudate measurement based on the size parameter, code for identifying, using the processing unit, an exudate area within the acquired image, and code for calculating, using the processing unit, a spread of exudate in the wound dressing based on the exudate area and the determined scale. Also this aspect of the present disclosure provides similar advantages as discussed above in relation to the previous aspects of the present disclosure.

A software executed by the processing unit for operation in accordance to the present disclosure may be stored on a computer readable medium, being any type of memory device, including one of a removable non-volatile random access memory, a hard disk drive, a floppy disk, a CD-ROM, a DVD-ROM, a USB memory, an SD memory card, a solid state drive, other non-volatile flash based storage media, or a similar computer readable medium known in the art.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Fig. 1 conceptually illustrates a computer system according to a currently preferred embodiment of the present disclosure,
Fig. 2 presents an exemplary wound dressing for use in relation to the computer system of claim 1,
Fig. 3 exemplary illustrates operation of the computer system, and
Fig. 4 is a flow charts illustrating the steps of performing the method according to a currently preferred embodiment of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

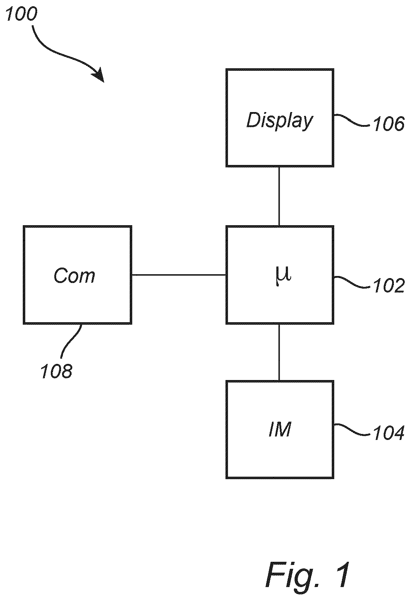

Turning now to the drawings and to Fig. 1 in particular, there is conceptually illustrated a computer system 100, presented in the form of a portable electronic user device intended to be operated by a user, such as a healthcare professional or a patient. The electronic user device may, for example, be implemented as a smartphone, tablet, or a purpose-specific medical imaging device.

The computer system 100 comprises a processing unit 102, an imaging device 104, and a display unit 106. The processing unit 102 is operatively connected to the imaging device 104 and is adapted to control its operation, including initiating image capture of a wound dressing. The processing unit 102 is further adapted to receive the captured image from the imaging device 104 and to process the image in accordance with the method steps described herein. In some embodiments, the processing unit 102 is also configured to extract data from an identifier associated with the wound dressing, and to perform calculations required for determining exudate spread.

For reference, the processing unit 102 may for example be manifested as a general-purpose processor, a graphics processing unit, an application specific processor, a circuit containing processing components, a group of distributed processing components, a group of distributed computers configured for processing, a field programmable gate array (FPGA), etc. The processor may be or include any number of hardware components for conducting data, signal and/or image processing or for executing computer code stored in memory. It may also be possible and within the scope to make use of system-on-chip (SOC) implementations. The memory may be one or more devices for storing data and/or computer code for completing or facilitating the various methods described in the present description. The memory may include volatile memory or non-volatile memory. The memory may include database components, object code components, script components, or any other type of information structure for supporting the various activities of the present description. According to an exemplary embodiment, any distributed or local memory device may be utilized with the systems and methods of this description. According to an exemplary embodiment the memory is communicably connected to the processor (e.g., via a circuit or any other wired, wireless, or network connection) and includes computer code for executing one or more processes described herein.

The display unit 106 is configured to present a graphical user interface (GUI) to the user. The GUI may be used to initiate or guide image acquisition and present processed results such as the calculated exudate spread, alerts, or wound trend visualizations. In some implementations, the GUI may also provide interactive functionality, such as allowing the user to adjust the framing manually or confirm automatic framing results.

Optionally, the computer system 100 may comprise a network communication module 108, which is configured to enable wireless communication with external systems or remote processing infrastructure. The communication module 108 may support standard protocols and interfaces, such as Wi-Fi, cellular data (e.g., LTE, 5G), or Bluetooth, allowing the system to transmit acquired images, metadata, or identifier information to an external processing unit.

In some embodiments, the system may be arranged such that certain computational steps are performed externally by a remote server (not shown). In such an arrangement, the portable electronic user device functions primarily as a front-end for image capture and user interaction, while the heavier computation tasks are offloaded to the server. The server may then transmit the analysis results back to the device for display to the user via the GUI. This client-server architecture may be beneficial in scenarios where the local device has limited processing capability or where centralized analysis is preferred for standardization or data aggregation. In further variations, the computer system 100 may be configured for hybrid operation, wherein certain steps (e.g., identifier recognition, initial framing) are performed locally, and others (e.g., AI-based refinement or trend analysis) are handled by the server. Such a division allows for flexible deployment based on clinical setting, device type, or connectivity availability.

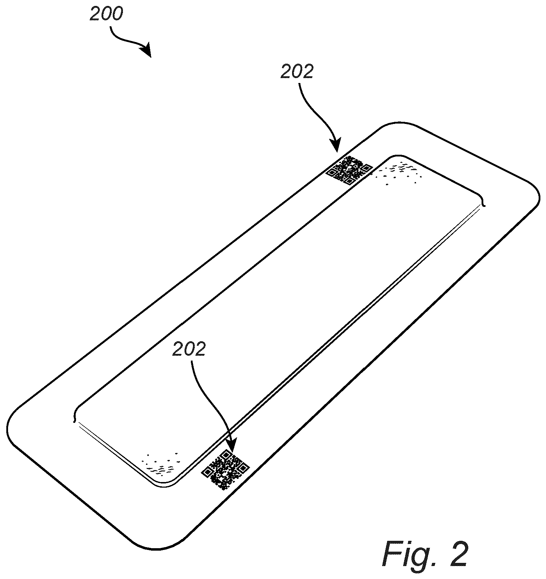

Turning now to Fig. 2, there is conceptually illustrated a wound dressing 200 suitable for use in connection with the computer system 100 illustrated in Fig. 1. The wound dressing 200 typically configured to be placed over a wound site on the skin of a patient to absorb wound exudate during the healing process.

In line with the present disclosure, the wound dressing 200 comprises a first identifier 202. In the illustrated embodiment, the first identifier 202 is implemented as a machine-readable code in the form of a QR code, arranged on an outer side of the wound dressing 200. The outer side is defined as the surface of the dressing facing away from the wound when the dressing is applied to the skin. Such placement allows the first identifier 202 to be scanned or read without removing or disturbing the dressing, thereby supporting non-invasive measurements.

In the embodiment illustrated in Fig. 2, an optional second identifier 202 may also be provided on the wound dressing 200. The second identifier 202 is shown as a QR code positioned diagonally relative to the orientation of the first identifier 202. The inclusion of such a second identifier 202 is intended to improve robustness in practical use, for example by facilitating scanning or detection regardless of how the dressing is oriented on the patient or how the electronic user device is positioned during imaging. In some scenarios, depending on patient posture, lighting, or anatomical placement, one identifier may be obscured or otherwise difficult to scan. The presence of a second, redundantly placed identifier mitigates such challenges and contributes to a more user-friendly and reliable workflow. It should be appreciated that inclusion of the second identifier 202 is optional, and may be employed as needed to support flexible scanning in varied clinical environments.

While the embodiment illustrated in Fig. 2 shows the identifier 202 as a QR code printed at a surface of the wound dressing 200, other implementations are also contemplated. For example, the identifier 202 may alternatively be a barcode or an NFC tag, or any other form of data-encoded element that may be scanned or accessed using an imaging or communication device. In some embodiments, the identifier 202 may be visually printed, laser-marked, or embedded into a label material bonded to the outer surface of the dressing.

It should be appreciated that the exact position of the identifier 202 is not limited to the wound dressing 200 itself. In certain embodiments, the identifier 202 may instead be provided on a separate label, tag, or packaging component accompanying the wound dressing 200, such as on a sterile pouch, peel-off backing, or product insert. When the identifier is provided separately, the information encoded in the identifier is still uniquely associated with the particular wound dressing 200 and may be scanned prior to application or as part of the initial setup phase in the wound monitoring process.

In use, the identifier 202 may provide or reference one or more parameters associated with the wound dressing 200, including but not limited to a size parameter or an absorption capacity parameter. The identifier 202 may either contain the relevant parameter directly or act as a pointer to an external data source, such as a database or product registry, from which the system may retrieve dressing-specific metadata.

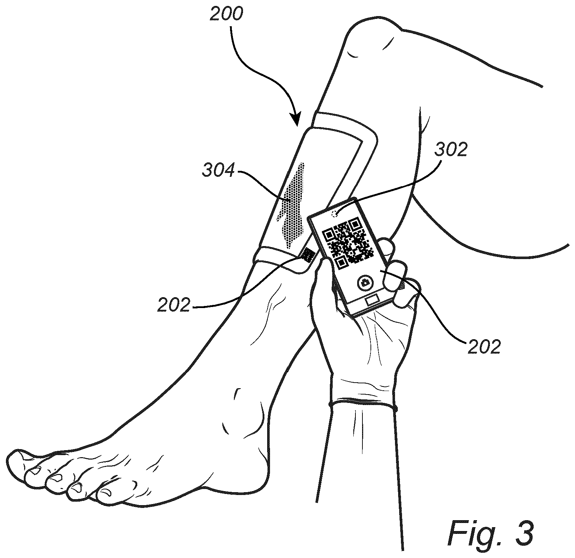

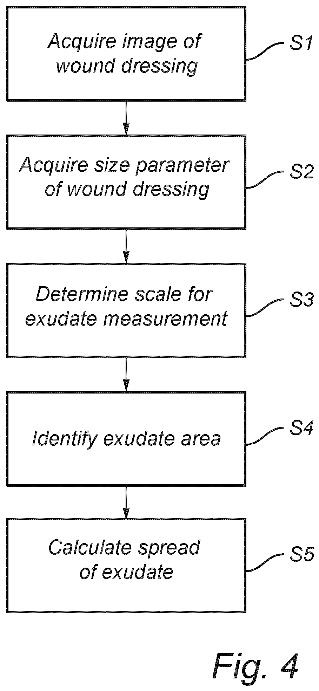

Turning now to Fig. 3 in conjunction with Fig. 4, there is illustrated, by way of example, the operation of the computer system 100 in conjunction with the wound dressing 200, and the steps of a method according to a currently preferred embodiment of the present disclosure.

In use, a user 302 (such as the above-mentioned healthcare professional or patient) is provided with access to a mobile electronic user device, for example implemented as the computer system 100 described in Fig. 1. In Fig. 3, the wound dressing 200 has already been applied to a wound site on the skin of a patient 304 and includes an associated identifier 202, such as a machine-readable code. As further illustrated, the wound dressing 200 is affected by exudate 306 at an area of the wound dressing 200, which has been absorbed and visibly spread within the dressing material of the wound dressing 200.

The process starts by the user 302 acquiring, S1, an image of the wound dressing 200 using the imaging device 104 of the computer system 100. The image may include both the visible area of the dressing and the identifier 202, provided on the outer surface of the dressing or separately on associated packaging. In some embodiments, the imaging device 104 may be configured to apply contrast enhancement, edge detection, or auto-focus adjustments to optimize the image for subsequent analysis. Metadata such as timestamp, illumination level, or camera orientation may also be captured and stored alongside the image for quality control or contextual processing.

In some embodiments, the acquired image may comprise a standard visible-spectrum photograph. However, it may also be possible to include additional spectral channels, such as near-infrared or multispectral imaging data, depending on the capabilities of the imaging device 104. Such extended imaging modalities may assist in improving the robustness of exudate detection, particularly in cases where contrast between the exudate and dressing material is limited under standard lighting conditions. The inclusion of spectral diversity may further benefit artificial intelligence-based analysis by increasing feature visibility and enabling more nuanced classification.

Additionally, the processing unit 102 is operated to acquire, S2, a size parameter of the wound dressing 200 based on data obtained from the identifier 202. As previously described, the identifier 202 may directly contain the size parameter or point to an external data source from which the size parameter is retrieved. Once the size parameter is available, the processing unit 102 proceeds to determine a scale for exudate measurement based on this parameter. The size parameter may, for example, represent a predefined nominal dimension of the dressing (e.g., 10 cm × 15 cm) or a calibration value. The scale may be used to convert measurements within the image (e.g., in pixels) to real-world dimensions (e.g., square centimeters), enabling accurate quantification of exudate spread. In some embodiments, a product code extracted from the identifier may be used to query a cloud-based registry of dressing specifications from which both size and absorption capacity parameters are retrieved.

Thereafter, the method continues by identifying, S3, an exudate area corresponding to the exudate 304 within the acquired image, where the processing unit 102 identifies the exudate area through image analysis, either rule-based or enhanced by artificial intelligence techniques trained to detect visual features characteristic of the wound exudate 306, such as texture, contrast, or edge boundaries. The identification process may include thresholding algorithms, color segmentation, or texture mapping to distinguish the exudate region from the surrounding dressing material. In some embodiments, the image may be overlaid with a bounding box or contour to visually indicate the detected area. Manual input may optionally be used to confirm or adjust the identified region via the GUI, providing flexibility for clinical workflows.

Following identification, the method proceeds to calculate, S4, a spread of exudate in the wound dressing 200 based on the exudate area and the determined scale. The calculated spread may represent an absolute surface area affected by exudate, a percentage of the total dressing area, or a categorized severity level. The processing unit 102 may optionally compare the calculated spread against predefined thresholds associated with the dressing type or wound condition, triggering alerts or recommendations if necessary. The result may be presented within the GUI of the computer system 100, stored in a patient database, or used as input for further downstream actions.

In some implementations, the processing unit 102 may also be configured to retrieve patient-specific information (such as wound type, wound location, or relevant medical history) from a local memory, external electronic health record system, or manual input via the GUI. This information may be used to adapt the processing logic, for example by adjusting the threshold values used in alert generation or trend classification. By incorporating patient context, the accuracy and clinical relevance of the measured exudate spread can be further improved, supporting individualized wound care management.

As illustrated in Fig. 3, the process may involve further interaction between the local device and a remote processing server, in cases where the scale determination, exudate identification, or calculation steps are offloaded for remote execution. In such implementations, the imaging and identifier acquisition are performed locally, while the remaining steps are computed remotely and returned to the user device for display and storage.

Furthermore, the control functionality of the present disclosure may be implemented using existing computer processors, or by a special purpose computer processor for an appropriate system, incorporated for this or another purpose, or by a hardwire system. Embodiments within the scope of the present disclosure include program products comprising machine-readable medium for carrying or having machine-executable instructions or data structures stored thereon. Such machine-readable media can be any available media that can be accessed by a general purpose or special purpose computer or other machine with a processor. By way of example, such machine-readable media can comprise RAM, ROM, EPROM, EEPROM, CD-ROM or other optical disk storage, magnetic disk storage or other magnetic storage devices, solid state drives or other non-volatile flash based storage devices, or any other medium which can be used to carry or store desired program code in the form of machine-executable instructions or data structures and which can be accessed by a general purpose or special purpose computer or other machine with a processor. When information is transferred or provided over a network or another communications connection (either hardwired, wireless, or a combination of hardwired or wireless) to a machine, the machine properly views the connection as a machine-readable medium. Thus, any such connection is properly termed a machine-readable medium. Combinations of the above are also included within the scope of machine-readable media. Machine-executable instructions include, for example, instructions and data which cause a general-purpose computer, special purpose computer, or special purpose processing machines to perform a certain function or group of functions.

Although the figures may show a sequence the order of the steps may differ from what is depicted. Also two or more steps may be performed concurrently or with partial concurrence. Such variation will depend on the software and hardware systems chosen and on designer choice. All such variations are within the scope of the disclosure. Likewise, software implementations could be accomplished with standard programming techniques with rule-based logic and other logic to accomplish the various connection steps, processing steps, comparison steps and decision steps. Additionally, even though the present disclosure has been described with reference to specific exemplifying embodiments thereof, many different alterations, modifications and the like will become apparent for those skilled in the art.

In addition, variations to the disclosed embodiments can be understood and effected by the skilled addressee in practicing the claimed present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A computer-implemented method for measuring exudate spread in a wound dressing, the method comprising the steps of:
acquiring, using a processing unit, an image of a wound dressing,
acquiring, using the processing unit, a size parameter of the wound dressing based on data obtained from an identifier associated with the dressing,
determining, using the processing unit, a scale for exudate measurement based on the size parameter,
identifying, using the processing unit, an exudate area within the acquired image, and
calculating, using the processing unit, a spread of exudate in the wound dressing based on the exudate area and the determined scale.

2. The method according to claim 1, wherein the identifier is a machine-readable code.

3. The method according to claim 2, wherein the machine-readable code is provided on an outer surface of the wound dressing, the outer surface being away from the wound

4. The method according to any one of claims 2 and 3, wherein the machine-readable code is selected from a QR code, barcode, or an NFC tag.

5. The method according to any one of the preceding claims, wherein the image of the wound dressing is acquired using an imaging device communicatively connected to the processing unit.

6. The method according to any one of the preceding claims, further comprising the step of acquiring, using the processing unit, an absorption capacity parameter of the wound dressing based on the identifier, wherein the calculated spread of exudate is correlated with the absorption capacity parameter.

7. The method according to any one of the preceding claims, further comprising the step of monitoring the exudate spread over time by periodically acquiring images of the wound dressing and comparing the calculated spread of exudate in the periodically acquired images.

8. The method according to any one of the preceding claims, further comprising the step of framing the exudate area within the acquired image, wherein the framing is performed either manually by a user or automatically using the processing unit.

9. The method according to any one of the preceding claims, further comprising the steps of:
generating, using the processing unit, an alert for a healthcare professional when a threshold level of exudate spread is detected based on the calculated spread of exudate, and
providing, using the processing unit, the alert within a graphical user interface (GUI) of an electronic user device.

10. The method according to any one of the preceding claims, wherein the identifier provides a pointer to an external source containing at least one of the size parameter and the absorption capacity parameter of the wound dressing.

11. The method according to any one of the preceding claims, further comprising the steps of storing the acquired images and calculated exudate spread values in a patient record database for continuous monitoring.

12. A computer system for measuring exudate spread in a wound dressing, wherein the computer system comprises a processing unit adapted to:
acquire an image of a wound dressing,
acquire a size parameter of the wound dressing based on data obtained from an identifier associated with the dressing,
determine a scale for exudate measurement based on the size parameter,
identify an exudate area within the acquired image, and
calculate a spread of exudate in the wound dressing based on the exudate area and the determined scale.

13. The computer system according to claim 12, wherein the computer system is a handheld electronic device, further comprising a display screen for presenting a graphical user interface (GUI) and an imaging device configured to acquire the image of the wound dressing.

14. The computer system according to any one of claims 12 and 13, wherein the processing unit is communicatively connected to a remote database, the remote database containing data associated with the identifier, including at least one of the size parameter and an absorption capacity parameter of the wound dressing.

15. A computer program comprising instructions which, when executed by a computer or processor, cause a computer system to measure exudate spread in a wound dressing, wherein the computer system comprises a processing unit, and wherein the computer program comprises:
code for acquiring, using a processing unit, an image of a wound dressing,
code for acquiring, using the processing unit, a size parameter of the wound dressing based on data obtained from an identifier associated with the dressing,
code for determining, using the processing unit, a scale for exudate measurement based on the size parameter,
code for identifying, using the processing unit, an exudate area within the acquired image, and
code for calculating, using the processing unit, a spread of exudate in the wound dressing based on the exudate area and the determined scale.
